(19) Europäisches Patentamt
European Patent Office
Office européen des brevets

(11) Veröffentlichungsnummer: **0 103 817 B1**

# (12) EUROPÄISCHE PATENTSCHRIFT

(45) Veröffentlichungstag der Patentschrift: **26.10.88**

(51) Int. Cl.4: **C 07 D 277/68**

(21) Anmeldenummer: **83108845.5**

(22) Anmeldetag: **08.09.83**

## (54) Verfahren zur Herstellung von 2-Chlorbenzthiazol.

(30) Priorität: **17.09.82 DE 3234530**

(43) Veröffentlichungstag der Anmeldung: **28.03.84 Patentblatt 84/13**

(45) Bekanntmachung des Hinweises auf die Patenterteilung: **26.10.88 Patentblatt 88/43**

(84) Benannte Vertragsstaaten: **BE CH DE FR GB IT LI**

(56) Entgegenhaltungen:
**EP - A - 0 039 905**
**EP - A - 0 043 013**
**DE - A - 1 670 453**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Steffan, Guido, Dr., Im Herzogenfeld 52, D-5068 Odenthal (DE)**

EP 0 103 817 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 2-Chlorbenzthiazol durch Chlorierung von Benzthiazol.

2-Chlorbenzthiazol wird üblicherweise hergestellt durch Umsetzung von 2-Mercaptobenzthiazol mit Chlor, Schwefelchloriden, Thionylchlorid, Phosgen, Sulfurylchlorid oder ähnlichen Chlorierungsmitteln (vgl. Houben-Weyl, Methoden der Organischen Chemie, 4. Auflage, Band 5/3, Seiten 858 und 871 (1962); ferner J. Amer. Chem. Soc. 68, S. 1666 (1946); DE-AS 1 164 413).

Diese Verfahren führen jedoch nicht zu befriedigenden Ausbeuten, ausserdem entstehen wenig brauchbare Nebenprodukte. Bei der Verwendung von Phosgen als Reagens erhält man zwar gute 2-Chlorbenzthiazol-Ausbeuten (DE-AS 1 164 413), aber das kaum verwendbare Kohlenoxysulfid als Nebenprodukt.

Weiterhin ist bekanntgeworden, dass man 2-Chlorbenzthiazol erhält, wenn man 2-Mercaptobenzthiazol oder dessen Disulfid mit Chlor in Gegenwart von inerten organischen Lösungs- oder Verdünnungsmitteln bei erhöhter Temperatur umsetzt (DE-A-1 670 453); als Nebenprodukt fällt Schwefeldichlorid an.

In einer neueren Patentanmeldung (EP-A1-0 039 905) wird weiterhin eine Methode zur Herstellung von 2-Chlorbenzthiazolen durch Umsetzung von 2-Chlorphenylisocyaniddichloriden mit Schwefel beschrieben; das benötigte Ausgangsprodukt muss jedoch zunächst hergestellt werden.

Ferner wurde ein Verfahren zur Herstellung von 2-Chlorbenzthiazol vorgeschlagen, welches dadurch gekennzeichnet ist, dass man 2-Hydrazinobenzthiazol mit Thionylchlorid umsetzt (EP-A-0 043 013). Hierbei muss das als Ausgangsprodukt zu verwendende 2-Hydrazino-benzthiazol zunächst erst hergestellt werden; als Nebenprodukte fallen Stickstoff, Schwefel, Schwefeldioxid und Chlorwasserstoff an.

Für eine grosstechnische Herstellung von 2-Chlorbenzthiazol erscheint die direkte Chlorierung des grosstechnisch verfügbaren Benzthiazols als besonders attraktiv.

Literatur oder Patente zur direkten Chlorierung von Benzthiazol zu 2-Chlorbenzthiazol konnten nicht aufgefunden werden. Demnach scheint die direkte Chlorierung von Benzthiazol zu 2-Chlorbenzthiazol bisher nicht gelungen zu sein.

In Ullmann's «Enzyklopädie der technischen Chemie», 3. Auflage, Band 17, Seite 331, heisst es: «Beim Benzothiazol treten die Substituenten (gemeint ist auch Halogen) in den Benzolring, und zwar bevorzugt in 6- oder 4-Stellung ein.»

Diese Feststellung kann bei üblicher Chlorierung auf Grund eigener Versuche bestätigt werden: Es entsteht ein Gemisch chlorierter Produkte, dessen Hauptkomponenten das Chlor am Benzolring tragen und das nur schwierig aufzutrennen ist.

Überraschenderweise wurde nun gefunden, dass man 2-Chlorbenzthiazol durch direkte Chlorierung von Benzthiazol in hohen Ausbeuten erhält, wenn man Benzthiazol in einem inerten, siedenden Lösungsmittel in Gegenwart von Eisen-III-chlorid oder Aluminiumchlorid als Katalysator und gegebenenfalls in Gegenwart von Phosphortrichlorid oder Pyridin als Co-Katalysator mit elementarem Chlor chloriert. Geeignete Lösungsmittel sind Phosphoroxychlorid ($POCl_3$) und insbesondere Chlorbenzol.

Unter den erfindungsgemässen Bedingungen tritt das Chlor fast ausschliesslich in die gewünschte 2-Stellung des Heterocyclus ein. Die Struktur von 2-Chlorbenzthiazol wurde durch Elementaranalyse, IR-Spektrum, NMR-Spektrum und Bestimmung des verseifbaren Chlors bestätigt.

Das Verfahren der Erfindung sei anhand folgender Reaktionsgleichung näher erläutert:

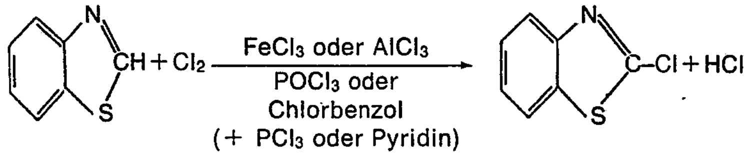


Bei der Durchführung des erfindungsgemässen Verfahrens setzt man auf 1 Mol Benzthiazol mindestens 1 Mol Chlor ein. Zur Erzielung eines möglichst vollständigen Umsatzes ist es jedoch zweckmässig, Chlor in leichtem Überschuss, zum Beispiel in einem bis zu 20%igen Überschuss, einzusetzen. Bevorzugt wird die ca. 1,1-fache stöchiometrisch erforderliche Menge Chlor zur Reaktion eingesetzt.

Das als Katalysator dienende Aluminiumchlorid oder Eisen-III-chlorid wird jeweils in Mengen von 0,2 bis 2 Mol-%, bezogen auf Benzthiazol, eingesetzt.

Der Start der Chlorierung kann wesentlich erleichtert werden dadurch, dass man dem Reaktionsgemisch vor Beginn der Chlorierung einen der genannten Co-Katalysatoren zusetzt; die eingesetzte Menge sollte etwa der Menge an Katalysator entsprechen.

Ausserdem ist es zweckmässig, ein bestimmtes Programm der Chloreinleitung einzuhalten: zu Beginn soll Chlor recht langsam, später zügig und gegen Ende wieder langsamer eingeleitet werden.

Bei dieser Verfahrensweise wird 2-Chlorbenzthiazol in Ausbeuten von über 90% d.Th. erhalten

und lässt sich durch Destillation mit Gehalten von 97 bis über 99% gewinnen.

Es zeigt sich, dass bei stärkerer Verdünnung mit Lösungsmittel die Ausbeuten tendenziell besser werden; wird eine bestimmte Lösungsmittelmenge unterschritten, so fallen die Ausbeuten stark ab. Am zweckmässigsten arbeitet man mit Lösungsmittelmengen von 180 bis 300 ml Phosphoroxychlorid bzw. von 200 bis 350 ml Chlorbenzol pro Mol Benzthiazol. Bemerkenswert ist, dass das Chlorbenzol unter den beschriebenen Reaktionsbedingungen nicht zu höherchlorierten Benzolen weiterchloriert wird.

Die Reaktionstemperatur ist durch die Siedetemperatur des verwendeten Lösungsmittels gegeben. Im allgemeinen arbeitet man bei Normaldruck.

Das 2-Chlorbenzthiazol wird aus dem Reaktionsgemisch in üblicher Weise destillativ abgetrennt.

2-Chlorbenzthiazol kann z.B. als Zwischenprodukt zur Herstellung von bestimmten Herbiziden verwendet werden (vgl. z.B. EP-B1-0 005 501). So kann durch Umsetzung mit Hydroxyessigsäure-N-methylanilid der bekannte herbizide Wirkstoff Benzthiazol-2-yloxyessigsäure-N-methyl-anilid hergestellt werden (vgl. auch EP-A1-0 014 409).

Die nachfolgenden Beispiele sollen zur weiteren Erläuterung der Erfindung dienen. (Die Abkürzung GC steht für Gaschromatogramm.)

Beispiele

Beispiel 1

In eine Mischung von 400 ml Phosphoroxychlorid, 270 g (2 Mol) Benzthiazol, 1,7 g Aluminiumchlorid und 1,7 g Pyridin werden bei Siedetemperatur (115 °C) innerhalb von 3 Stunden 150 g Chlor eingeleitet (starke HCl-Entwicklung).

Das Reaktionsgemisch wird 1 Stunde nachgerührt und anschliessend über eine kurze Vigreux-Kolonne destillativ – teilweise im Vakuum – aufgetrennt. Man erhält:

638 g Vorlauf (nach GC: 99,6% $POCl_3$, 0,4% Benzthiazol)
316 g Hauptlauf (nach GC: 0,15% $POCl_3$,
0,1% Benzthiazol,
98,3% 2-Chlorbenzthiazol,
1,4% Unbekannte) und
34 g Destillationsrückstand.

Ausbeute an 2-Chlorbenzthiazol: 91,6% d. Th.

Beispiel 2

In eine Mischung von 1000 kg Phosphoroxychlorid, 405 kg (3 KMol) Benzthiazol, 3 kg Eisen-III-chlorid und 10 kg Phosphortrichlorid werden bei 115 °C (Rückfluss) 8 kg Chlor eingeleitet.

Man lässt 15 Minuten nachrühren und leitet dann bei kräftigem Rückfluss weitere 232 kg Chlor nach folgendem Programm ein:

1. Stunde: 40 kg Chlor
2. Stunde: 80 kg Chlor
3. Stunde: 80 kg Chlor
4. Stunde: 32 kg Chlor

(Starke HCl-Entwicklung)

Man lässt dann 1 Stunde bei 115 °C nachrühren und destilliert anschliessend das Phosphoroxychlorid über eine kurze Kolonne ab, zunächst bei Normaldruck, zuletzt in zunehmendem Vakuum.

Danach wird bei 20 mbar und Kopftemperaturen von 122–125 °C 2-Chlorbenzthiazol abdestilliert.

Man erhält 478 kg Hauptlauf mit einem Gehalt von 97,8% 2-Chlorbenzthiazol, was einer Ausbeute von 91,9% der Theorie entspricht.

Der Destillationsrückstand von ca. 43 kg ist gut in 1,2-Dichlorbenzol löslich.

Beispiel 3

In eine Mischung aus 585 ml Chlorbenzol, 270 g Benzthiazol (2 Mol), 2 g Eisen-III-chlorid und 7 g Phosphortrichlorid werden bei 100 °C 10 g Chlor eingeleitet.

Bei Rückflusstemperatur werden dann in 5–6 Stunden weitere 140 g Chlor – anfangs recht langsam, später zügig – eingeleitet.

Nach einstündigem Nachrühren ist die Gasentwicklung zu Ende.

Nach üblicher destillativer Aufarbeitung im Vakuum wurden erhalten:

624 g Chlorbenzol (mit 1,1% Benzthiazol und 2,1% 2-Chlor-benzthiazol),
312 g Hauptlauf (0,8% Chlorbenzol,
98,6% 2-Chlorbenzthiazol,
0,7% Unbekannte)
und
29 g Destillationsrückstand.

Ausbeute an 2-Chlorbenzthiazol:
95,0% d.Th., bezogen auf Einsatz;
(97,5% d.Th., bezogen auf Umsatz).

Bei Verwendung von nur 450 ml Chlorbenzol statt 585 ml wird nur eine Ausbeute von 90,1% d.Th. erhalten; bei Verwendung von nur 360 ml sogar nur 71,7% d.Th. an 2-Chlorbenzthiazol.

Verwendung einer äquimolaren Menge Aluminiumchlorid an Stelle von Eisen-III-chlorid ist mit praktisch gleichem Erfolg möglich.

**Patentansprüche**

1. Verfahren zur Herstellung von 2-Chlorbenzthiazol durch direkte Chlorierung von Benzthiazol, dadurch gekennzeichnet, dass man Benzthiazol in einem inerten, siedenden Lösungsmittel in Gegenwart von Eisen-III-chlorid oder Aluminiumchlorid als Katalysator und gegebenenfalls in Gegenwart von Phosphortrichlorid oder Pyridin als Co-Katalysator mit elementarem Chlor chloriert.

2. Verfahren nach Anspruch 1, dadurch gekennzeichnet, dass man als Lösungsmittel Phosphoroxychlorid oder Chlorbenzol verwendet.

**Claims**

1. Process for the preparation of 2-chlorobenzthiazole by direct chlorination of benzthiazole, characterised in that benzthiazole is chlorinated with elementary chlorine in an inert, boiling solvent in the presence of iron-III chloride or aluminium chloride as a catalyst and, if appropriate, in

the presence of phosphorus trichloride or pyridine as a co-catalyst.

2. Process according to Claim 1, characterised in that the solvent used is phosphorus oxychloride or chlorobenzene.

**Revendications**

1. Procédé de fabrication du 2-chlorobenzothiazol par chloruration directe du benzothiazol, caractérisé en ce qu'on chlore avec du chlore élémentaire le benzothiazol dans un solvant inerte bouillant en présence de chlorure de fer-III ou de chlorure d'aluminium comme catalyseur et éventuellement en présence de trichlorure de phosphore ou de pyridine comme co-catalyseur.

2. Procédé selon la revendication 1, caractérisé en ce qu'on utilise de l'oxychlorure de phosphore ou du chlorobenzène comme solvant.